(19) Europäisches Patentamt
European Patent Office
Office européen des brevets

(11) Publication number: 0 092 786 B1

(12) **EUROPEAN PATENT SPECIFICATION**

(45) Date of publication of patent specification: **07.01.87**

(21) Application number: **83103839.3**

(22) Date of filing: **20.04.83**

(51) Int. Cl.⁴: **C 07 D 471/04,**
C 07 D 213/78,
C 07 D 241/26, A 61 K 31/44,
A 61 K 31/505 // (C07D471/04, 221:00, 221:00),(C07D471/04, 241:00, 221:00)

(54) **1,8-Naphthyridine and 1,5,8-azanaphthyridine derivatives.**

(30) Priority: **26.04.82 US 371623**
**03.11.82 US 438681**

(43) Date of publication of application:
**02.11.83 Bulletin 83/44**

(45) Publication of the grant of the patent:
**07.01.87 Bulletin 87/02**

(84) Designated Contracting States:
**AT BE CH DE FR GB IT LI NL SE**

(56) References cited:
**US-A-4 128 649**

**CHEMICAL ABSTRACTS, vol. 88, no. 15, April 10, 1978, pages 570-1, abstract 105298y, Columbus, Ohio, US**

(73) Proprietor: **SCHERING CORPORATION**
**2000 Galloping Hill Road**
**Kenilworth, New Jersey 07033 (US)**

(72) Inventor: **Sherlock, Margaret H.**
**34 Parkway West**
**Bloomfield New Jersey 07003 (US)**

(74) Representative: **Ritter, Stephen David et al**
**Mathys & Squire 10 Fleet Street**
**London EC4Y 1AY (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel 1,8-naphthyridine- and 1,5,8-aza-naphthyridine- derivatives of the general formula

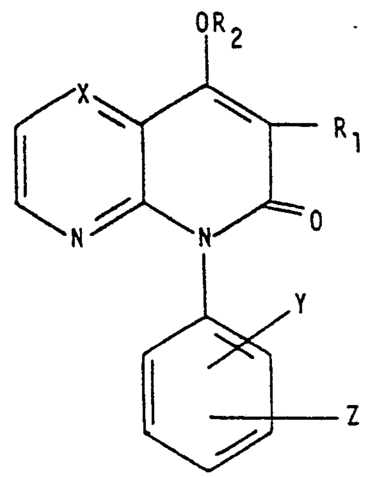

I

wherein

X is CH or N;

Y is hydrogen, hydroxy, benzyloxy, amino, sulfamyl, halogen, nitro, alkyl having from 1 to 6 carbon atoms, alkoxy having from 1 to 6 carbon atoms, carboxylic acyl having from 2 to 6 carbon atoms, alkyl-$S(O)_m$- having from 1 to 6 carbon atoms wherein m is 0, 1 or 2, trifluoromethyl, trifluoromethylthio, or COOA wherein A is hydrogen, alkyl having from 1 to 6 carbon atoms or a pharmaceutically acceptable cation derived from a metal or an amine;

Z is hydrogen, hydroxy, halogen, alkyl having from 1 to 6 carbon atoms, alkoxy having from 1 to 6 carbon atoms, hydroxy-alkyl having from 1 to 6 carbon atoms or carboxylic acyloxy having from 2 to 6 carbon atoms;

$R_1$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 4- or 5-pyrimidyl, 2-, 3- or 4-pyridyl, 2- or 3-thienyl, 2- or 3-furanyl, carboxylic acyl having from 2 to 6 carbon atoms, or alkyl having from 1 to 10 carbon atoms which may be substituted with hydroxy, halogen, alkoxy having from 1 to 6 carbon atoms, carboxylic acyl having from 2 to 6 carbon atoms, phenyl, 2-, 3- or 4-pyridyl, 2-, 4- or 5-pyrimidyl, 2- or 3-thienyl, 2- or 3-furanyl, cycloalkyl having from 3 to 7 carbon atoms or carboxylic acycloxy having from 1 to 6 carbon atoms.

$R_2$ is hydrogen, carboxylic acyl having from 1 to 6 carbon atoms, alkyl having from 1 to 6 carbon atoms, mono- or di-hydroxyalkyl having from 2 to 6 carbon atoms, alkenyl having from 2 to 6 carbon atoms, alkynyl having from 2 to 6 carbon atoms, $R_aR_bN(CH_2)_n$– wherein n is an integer of from 2 to 6 and $R_a$ and $R_b$ are hydrogen, alkyl having from 1 to 6 carbon atoms or form, together with the nitrogen atom to which they are attached, a piperidine, morpholine, piperazine or pyrrolidine ring or $R_2$ represents a pharmaceutically acceptable cation derived from a metal or an amine, with the proviso that when X is CH, $R_2$ is hydrogen or carboxylic acyl, Y is hydrogen, alkyl, alkoxy, $CF_3$, $CH_3SO_2$, —$SCF_3$ or $NO_2$ *and* Z is hydrogen, halogen, alkyl or alkoxy, *then* $R_1$ is not unsubstituted alkyl; the acid addition salts and hydrates and solvates of such compounds.

The 1,8-naphthyridines of Formula I, i.e. compounds wherein X is CH, are preferred.

The preferred meaning of Y is hydrogen, methylthio and alkoxy, and of Z, hydrogen or alkyl.

Preferred definitions for $R_1$ are alkenyl having 3 or 4 carbon atoms or ω-hydroxyalkyl having from 2 to 4 carbon atoms and, under the proviso defined above *n*-alkyl having from 3 to 5 carbon atoms and ω-carboxylicacyloxyalkyl.

As used herein, the terms alkyl, alkoxy, alkenyl, alkynyl etc., are meant to cover straight and branched chains. Halogen covers fluorine, chlorine, bromine and iodine. The cations represented by $R_2$ may be derived from any pharmaceutically acceptable metal, amine-metal and amine. Preferred are sodium, potassium, calcium, aluminium, N-methylglucamine, lysine, etc.

A most preferred group comprises compounds of Formula I wherein

Y is selected from hydrogen, methylthio and methoxy.

Z is hydrogen or methyl,

$R_1$ represents n-$CH_4H_9$, —$(CH_2)_3CH_2OCOC_2H_5$, —$(CH_2)_2CH_2OH$, —$CH_2{\cdot}CH_2{\cdot}OH$ or —$CH_2$—$CH = CH_2$ and

$R_2$ represents sodium cation, $—CH_2CH_2OH$ or, provided $R_1$ is not $n\text{-}C_4H_9$, also hydrogen, $—COCH_3$ or $—COC_2H_5$.

Particularly preferred compounds are:

4-acetoxy-1-phenyl-3-(2-propenyl)-1,8-naphthyridin-2(1*H*)-one (m.p. 195°—196°C).

4-hydroxy-1-(3-methoxyphenyl)-3-(2-propenyl)-1,8-naphthyridin-2(1*H*)-one (m.p. 200°C—201°C). and the sodium salt thereof; and the sodium salt of 4-hydroxy-1-phenyl-3-(n-butyl)-1,8-naphthyridin-2(1H)-one (m.p. 240°—260°).

The compounds of this invention are active for treating allergic reactions such as asthma, bronchitis and the like. Compounds of a related structure are know from the published Japanese patent application Nr. 116495/77 (Kokai) and US—A—4128649 to Sandoz Incorporated.

The compounds of said Japanese application are described as having analgesic, anti-inflammatory, central nervous system depressant and diuretic effect. Nowhere in the application is it indicated that the compounds have any anti-allergic properties. US—A—4128649 describes 4-hydroxy-pyridopyridine derivatives substituted in the 3-position by an optionally esterified carboxyl group. Such substituents are not present in the compounds of the invention.

The compounds of this invention may be prepared by methods known for the preparation of similar compounds, e.g. as described in the Japanese application referred to above.

Thus, the compounds of Formula I may be prepared by a process characterized in that:

A: a compound of the general Formula II

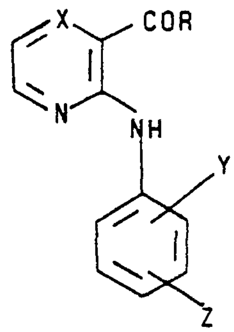

(II)

wherein X, Y and Z are as defined above and R represents any convenient leaving group, preferably an alkoxy group, is reacted with a compound of the general formula III

$$R_1CH_2COR \qquad III$$

wherein $R_1$ and R are as defined above; or

B: for the preparation of a compound of Formula I wherein $R_1$ represents an alkenyl group with 3 to 6 carbon atoms having the double bond in the 2,3-position, a compound of the general Formula IV

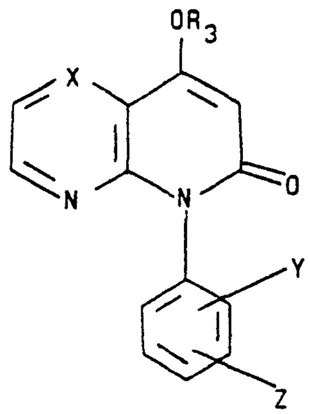

(IV)

wherein $R_3$ is an alkenyl group with 2 to 6 carbon atoms having the double bond in the 2,3-position, and X, Y and Z are as defined above, is subjected to a Claisen rearrangement, and that a compound obtained according to process A or B above wherein $R_2$ represents hydrogen, if desired, is subjected to introduction of a group $R_2$ different from hydrogen.

Reaction A is preferably carried out by bringing the reactants together in the presence of a base such as a metal alkoxide, e.g. potassium tertiary butoxide, at an elevated temperature, e.g. 60°C to 160°C. Preferably the reaction is carried out under an inert atmosphere such as nitrogen. Alternatively, the reaction may be carried out in the presence of a non-reactive solvent such as toluene, xylene, etc.

The starting compounds of Formulae II and III are either known compounds or they may be obtained according to processes well known in the art. The compounds of Formula II may be prepared according to standard procedures, e.g. as described in United States Reissue Patent Nr. 26, 655:

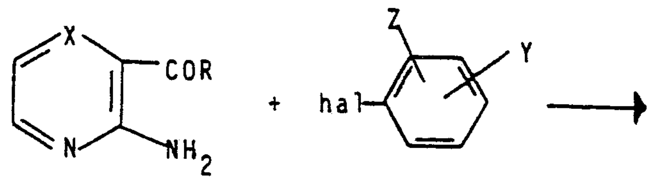

II

2-Anilino-3-pyrazine carboxylic acid is a known compound [C. A. *75,* 20154e (1971)], and the esters may be prepared by standard procedures. Equally, the compounds of Formula III are well known.

The Claisen rearrangement (process B) can be carried out simply be heating the starting alkenyl ether of Formula IV. The reaction may, however, also be carried out in a non-reactive high boiling solvent such as cymene. The resulting compound is then a compound of formula I wherein $R_2$ represents hydrogen. If the rearrangement is carried out in the presence of a carboxylic acid anyhdride, however, then a compound of Formula I wherein $R_2$ is a carboxylic acyl group is obtained directly.

The conversion of a compound of Formula I wherein $R_2$ is hydrogen into a compound wherein $R_2$ is different from hydrogen may be obtained by standard procedures such as acylation, alkylation and salt formation. Thus, for example, a compound wherein $R_2$ is 2-propenyl may be prepared by reacting a 4-hydroxy compound with allyl bromide. The acylation may be carried out according to well known methods, e.g. by reacting the 4-hydroxy compound with a reactive derivative (anhydride, halide) of a carboxylic acid under standard conditions.

As is obvious to anyone skilled in the art, certain groups represented by Y and Z or certain substituents in the groups $R_1$ and $R_2$ etc., may be interconverted, if desired, subsequent to the processes A and B. For example, a hydroxy group may be converted into halogen by treatment with a halogenating agent, e.g. thionylhalide etc.

Equally a compound having an acyl, ether or salt function in position 4 may be converted into the corresponding compound having a 4-hydroxy group by standard reactions. Thus, for example, a compound having a 4-acyl group may be treated with sodium hydroxide to yield a compound wherein $R_2$ is sodium. Further treatment of such compound with hydrochloric acid will yield a 4-hydroxy compound.

The compounds wherein $R_2$ represents a cation may easily be obtained by reacting the 4-hydroxy compound with an appropriate base or amine or reactive derivatives thereof.

### Example 1

4-acetoxy-1-phenyl-3-(2-propenyl)-1,8-naphthyridine-2(1H)-one

(A) 4-(2-propenyloxy)-1-phenyl-1,8-naphthyridin-2(1H)-one:

To a mixture of 62 g. of 4-hydroxy-1-phenyl-1,8-naphthyridin-2(1*H*)-one, 39.6 g. of anhydrous potassium carbonate and 1800 ml of acetone there is added dropwise with stirring, 37.5 g. of allyl bromide. The reaction mixture is refluxed for 22 hours, concentrated in vacuo, and the residue extracted with 600 ml. of chloroform. The organic extract is washed with water, 1N sodium hydroxide solution and again with water, dried over anhydrous magnesium sulfate, filtered and concentrated. The crude solid is triturated with 3 × 400 ml of boiling isopropyl ether, filtered, yielding the insoluble product, wt. 38.5 g. m.p. 171°—174°C. Recrystallization from methanol produces the product as a colourless solid, m.p. 176°—177°C.

(B) 4-acetoxy-1-phenyl-3-(2-propenyl)-1,8-naphthyridin-2(1H)-one

A mixture of 33.8 g. of 4-(2-propenyloxy)-1-phenyl-1,8-naphthyridin-2(1*H*)-one and 35 ml. of acetic anhydride is refluxed for four hours. On cooling, the reaction mixture soldifies. Trituration with isopropyl ether and filtration yields the product, 36.1 g., as a colourless solid, m.p. 189°—195°C. Recrystallization from ethanol provides the product of this example melting at 195°—196°C.

### Example 2

4-hydroxy-1-phenyl-3-(2-propenyl)-1,8-naphthyridin-2(1H)-one

A mixture of 6.0 g. of 4-acetoxy-1-phenyl-3-(2-propenyl)-1,8-naphthyridin-2(1*H*)-one, 200 ml. of ethanol and 40 ml. of 1N sodium hydroxide solution is stirred at room temperature for 22 hours. The ethanol is removed *in vacuo* and the remaining aqueous solution acidifed with 1N hydrochloric acid. The product is

filtered, washed with water and dried, weight 5.3 g., m.p. 248°—250°C. Recrystallization from chloroform yields the product of this example as a colourless solid, m.p. 250°—252°C.

Example 3

7-(N-butyl)-8-hydroxy-5-phenylpyrido[2,3-b]pyrazine 6(5H)-one

(A) Methyl-2-bromo-3-pyrazine carboxylate:

To a stirred mixture of 12.7 g. of methyl 2-amino pyrazine carboxylate and 47 ml. of 48% hydrobromic acid there is added, dropwise, 12.6 ml. of bromine keeping the temperature at 0°C. A solution of 14.4 g. of sodium nitrite in 60 ml. of water is then added, dropwise, at 0° and the reaction mixture stirred for 15 minutes. The reaction mixture is basified to pH 8 with sodium bicarbonate and extracted with ethyl acetate and again with chloroform. The organic layers are dried over magnesium sulfate, filtered and concentrated to a yellow oil. Recrystallization from ether-hexane yields the product, m.p. 43°—45°C.

(B) Methyl-2-phenylamino-3-pyrazine carboxylate:

A mixture of 9.5 g. of methyl 2-bromo-3-pyrazine carboxylate, 8.2 g. of aniline, 0.5 g. of p-toluene sulfonic acid and 100 ml. of water is stirred and refluxed for two hours. The reaction mixture is poured on ice, extracted with ethyl acetate, the organic extracts are dried and concentrated to yield an oil. The crude residue is eluted on a silica gel column with ethyl-acetate-hexane (1:2) yielding the product of this example as a yellow solid, m.p. 72°—75°C.

(C) 7(n-Butyl)-8-hydroxy-5-phenyl-pyrido[2,3-b]pyrazine-6(5H)-one:

A mixture of 3.5 g. of methyl-2-phenylamino-3-pyrazine carboxylate, 30 ml. of ethyl caproate and 4 g. of potassium tertiary butoxide is stirred and heated under nitrogen at 150°—160°C. for one and a half hours. The reaction mixture is poured on ice, extracted with ethyl acetate and the ethyl acetate extracts washed with water. The combined aqueous layers are acidified to pH 5.5 with dilute hydrochloric acid and the solid filtered. Recrystallization from ethyl acetate-hexane yields the product of this example as a colourless solid; m.p. 183°—185°C.

Example 4

3-(2-hydroxyethyl)-4-hydroxy-1-phenyl-1,8-Naphthyridin-2(1H)-one

To a solution of 6.8 g. of methyl-2-phenyl-amino-3-pyridine carboxylate in 60 ml. of γ-butyrolactone there is added, under nitrogen, 13.4 g. of potassium tertiary butoxide. The reaction mixture is heated and stirred for one hour at 95°C, poured on ice and stirred overnight. The mixture is extracted with ether, the aqueous layer acidified with acetic acid to pH 4.5 and the product is collected by filtration. Recrystallization from chloroform/acetone/isopropanol yields the product of this example as a coloureless solid; m.p. 235°—236°C.

Example 5

Sodium and potassium salt of 3-(N-butyl)-4-hydroxy-1-phenyl-1,8-Nyphthydrine-2(1H)-one

A: 3-(n-Butyl)-4-hydroxy-1-phenyl-1,8-naphthyridin-2(1H)-one

To a stirred solution of 9 g. of methyl 2-(phenylamino)-3-pyridine carboxylate in 90 ml. of ethyl caproate there is added, portionwise 7.26 g. of potassium tertiary butoxide in an atmosphere of nitrogen. The reaction mixture is heated to an internal temperature of 140—142°C., for two hours, cooled and the potassium salt is collected by filtration. The crude potassium salt is dissolved in 150 ml. of water, acidified with 10% hydrochloric acid and the product filtered and dried; weight 9.8 g., m.p. 238°—239°C.

B: 3-(n-Butyl)-4-hydroxy-1-phenyl-1,8-naphthyridine-2(1H)-one sodium salt

Add 57.9 ml. of 1N sodium hydroxide aqueous solution to a stirred suspension of 17.05 g. of 3-(n-butyl)-4-hydroxy-1-phenyl-1,8-naphthyridine-2(1*H*)-one and 140 ml of water. Stir for one hour at room temperature, cool in an ice bath and filter. Lyophilize the clear filtrate overnight to give the title compound as a monhydrate. It is a cream coloured powder, m.p. 240°—260°C (decomposition).

Prepare the corresponding potassium salt as a monohydrate by the process of Example 1 by replacing the sodium hydroxide aqueous solution with an equivalent amount of potassium hydroxide aqueous solution. The lyophilized potassium salt has a melting point of 215°—225°C.

Example 6

3-(n-butyl)-4-hydroxy-1-phenyl-1,8-naphthyridine-2-(1H)-one ethanolamine salt

Add 0.65 g. of ethanolamine to 3-(n-butyl)-4-hydroxy-1-phenyl-1,8-naphthyridine-2(1*H*)-one (2.95 g.) in 100 ml. of methanol. Remove the solvent *in vacuo* and add ethyl acetate to precipitate the title compound as a colourless salt, m.p. 228°—234°C.

Prepare the following amine salts by replacing the ethanolamine with the corresponding amine:

3-(n-Butyl)-4-hydroxy-1-phenyl-1,8-naphthyridine-2(1*H*)-one N-methylglucamine salt, melting point 181°—206°C.

3-(n-Butyl)-4-hydroxy-1-phenyl-1,8-naphthyridine-2(1*H*)-onė diethanolamine salt, melting point 160°—190°C.

3(n-Butyl)-4-hydroxy-1-phenyl-1,8-naphthyridine-2(1*H*)-one ethylenediamine salt, melting point 158°—171°C.

Example 7

3-(n-butyl)-4-hydroxy-1-phenyl-1,8-naphthyridine-2(1H)-one calcium salt

Treat a solution of 1 g (3 m moles) of the compound of Example 5A herein with 1.5 ml. of 1N calcium chloride solution (1.5 m moles). Filter the resulting cloudy solution and allow to stand until a precipitate forms. Filter and wash with acetone. Recover the title compound from water or acetone-water as a pentahydrate which is a colourless powder, m.p. 350°C.

In a similar manner is obtained:

4-hydroxy-3-(n-pentyl)-1-phenyl-1,8-naphtyridine-2(1*H*)-one sodium salt; m.p. of hydrate ($\frac{1}{2}H_2O$): 245°—270°C.

3-(n-butyl)-1-(2,6-dimethylphenyl)-4-hydroxy-1,8-naphthyridin-2(1*H*)-one sodium salt; m.p. of the dihydrate 270°—280°C.

According to the processes outlined and exemplified above the compounds of the following table may be obtained (X = CH):

| Compound No. | Y | Z | $R_1$ | $R_2$ | m.p. °C. |
|---|---|---|---|---|---|
| 1 | H | H | $-(CH_2)_3CH_3$ | $-CH_2CH_2N(CH_3)_2$ | of HCl-salt: 236–238 |
| 2 | H | H | $-(CH_2)_3CH_3$ | $-CH_2CH_2CH_2N(CH_3)_2$ | of HCl-salt: 197–199 |
| 3 | H | H | $-CH_2CH_2OH$ | H | 233–234 |
| 4 | H | H | $-(CH_2)_3CH_3$ | $-CH_2CH_2OH$ | 136–138 |
| 5 | H | H | $-(CH_2)_3CH_2OH$ | H | + 1/6 $CH_3CH(OH)CH_3$ (solvate) 205.5–206.5 |
| 6 | H | H | $-(CH_2)_2CH_2OH$ | H | 218–220 |
| 7 | H | H | $-CH_2CH(OH)CH_3$ | H | 236–238 |
| 8 | H | H | $-CH_2CH = CH_2$ | $-COCH_3$ | 195–196 |
| 9 | H | H | 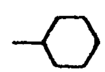 (cyclohexyl) | H | 300 |
| 10 | H | H | $-CH_2-CH(OH)-(CH_2)_3-CH_3$ | H | 176–178 |

| Compound No. | Y | Z | $R_1$ | $R_2$ | m.p. °C. |
|---|---|---|---|---|---|
| 11 | H | H | $-(CH_2)_3CH_3$ (X = N) | H | 183–185 |
| 12 | H | H | 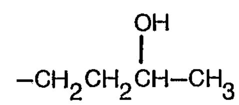 | H | 234–235 |
| 13 | H | H | $-CH_2CH=CH_2$ | H | 250–252 |
| 14 | H | H | 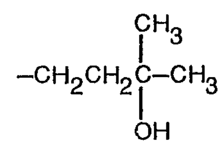 | H | 253–255 |
| 15 | H | H | $-CH_2CH_2CH_2CH_3$ | $-CH_2C\equiv CH$ | 102–104 |
| 16 | H | H | $-(CH_2)_3CH_2Br$ | H | 194–196 |
| 17 | H | H | $-(CH_2)_3CH_2Br$ | $-COCH_2CH_3$ | 147–149 |
| 18 | $4\text{-}OCH_3$ | H | $-(CH_2)_3CH_3$ | $-CH_2CH_2OH$ | 159–160 |
| 19 | H | H | $-(CH_2)_3CH_2Br$ | $-COCH_3$ | 168–170 |
| 20 | H | H | $-(CH_2)_3CH_3$ | $-CH_2CH=CH_2$ | 138–140 |

| Compound No. | Y | Z | $R_1$ | $R_2$ | m.p. °C. |
|---|---|---|---|---|---|
| 21 | H | H | $-(CH_2)_3CH_3$ | $-CH_2\underset{OH}{CH}CH_2OH$ | 162–163 |
| 22 | H | H | $-(CH_2)_3CH_3$ | $-CH_2CH_2CH_2OH$ | 173–175 |
| 23 | $4\text{-}OCH_3$ | H | $-CH_2CH=CH_2$ | H | 228–229 |
| 24 | H | $3\text{-}OCH_3$ | $-CH_2CH=CH_2$ | H | 200–201 (sodium salt: 220–235) |
| 25 | H | H | $-CH_2$ 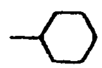 | H | 289–291 |
| 26 | H | H | $-CH_2CH_2$ 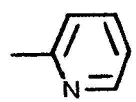 | H | 255–257 (dec.) |
| 27 | H | H | $-(CH_2)_3CH_2OH$ | H | 186–188 |
| 28 | H | H | $-CH_2(CH_2)_6CH=CH_2$ | H | 163–164 |
| 29 | H | H | $-CH_2(CH_2)_6CH=CH_2$ | $-COCH_3$ | 139–141 |
| 30 | $4\text{-}CH_3$ | H | $-(CH_2)_2CH_2OH$ | H | 227–228.5 |

| Compound No. | Y | Z | $R_1$ | $R_2$ | m.p. °C. |
|---|---|---|---|---|---|
| 31 | H | H | $-CH_2CH_2OH$ | H | 239–240.5 |
| 32 | H | 3-$OCH_3$ | $-CH_2CH_2OH$ | H | 1/4 $H_2O$ (hydrate): 228–229.5 |
| 33 | 4-$CH_3$ | 3-$OCH_3$ | $-CH_2CH_2OH$ | H | 1/4 $H_2O$: 217–219.5 |
| 34 | 4-$OCH_3$ | H | $-CH_2CH_2OH$ | H | 214–215 |
| 35 | 4-Cl | H | $-(CH_2)_2CH_2OH$ | H | 249.5–251 |
| 36 | H | H | $-(CH_2)_3CH_2-$ 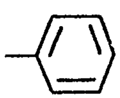 | H | 198–199 |
| 37 | 4-$OCH_3$ | H | $-(CH_2)_3CH_2OH$ | H | 237–239 |
| 38 | H | H | $-(CH_2)_3CH_3$ | $-CH_2CH_3$ | 171–172 |
| 39 | H | H | $-(CH_2)_3CH_2OCOCH_3$ | $-COCH_3$ | 161–162 |
| 40 | H | H | $-(CH_2)_3CH_2OCOCH_2CH_3$ | $-COCH_2CH_3$ | 119–120 |

| Compound No. | Y | Z | $R_1$ | $R_2$ | m.p. °C. |
|---|---|---|---|---|---|
| 41 | 4-$SCH_3$ | H | $-(CH_2)_3CH_3$ | H | 262–263 |
| 42 | H | H | $-(CH_2)_2CH_2-OCOCH_3$ | $-COCH_3$ | 173.5–175.5 |
| 43 | H | H | $-(CH_2)_2CH_2-OCOCH_2CH_3$ | $-COCH_2CH_3$ | 134–136.5 |
| 44 | H | H | $-CH_2CH_2OCOCH_2CH_3$ | $-COCH_2CH_3$ | 169–171.5 |
| 45 | 3-$SCH_3$ | H | $-(CH_2)_3CH_3$ | H | 245–246 |
| 46 | H | H | $-CH_2-CH-CH_3$<br>$OCOCH_3$ | $-COCH_3$ | 189–192 |
| 47 | H | H | $-CH_2-CH-CH_3$<br>$OCOCH_2CH_3$ | $-COCH_2CH_3$ | 182–184 |
| 48 | 4-$SCH_3$ | H | $-(CH_2)_3CH_3$ | $-COCH_3$ | 208–210 |
| 49 | H | H | $-CH_2-CH-(CH_2)_3-CH$<br>$OCOCH_2CH_3$ | $-COCH_2CH_3$ | 178–180 |

| Compound No. | Y | Z | $R_1$ | $R_2$ | m.p. °C. |
|---|---|---|---|---|---|
| 50 | H | H | $-CH_2-CH(CH_2)_3CH_3$<br>\|<br>$OCOCH_3$ | $-COCH_3$ | 177–178 |
| 51 | H | H | $-CH_2CH_2OCOCH_3$ | $-COCH_3$ | 183–185 |
| 52 | 4-$SOCH_3$ | H | $-(CH_2)_3CH_3$ | $-COCH_3$ | 218–219 |
| 53 | 3-$COOC_2H_5$ | 4-OH | $-(CH_2)_3CH_3$ | H | 194–195 |
| 54 | H | H | $-(CH_2)_3CH_2OCOCH(CH_3)_2$ | $-COCH(CH_3)_2$ | 145–146 |
| 55 | 3-COOH | 4-OH | $-(CH_2)_3CH_3$ | H | 274–276 (dec.) |
| 56 | 4-$OCH_2$— 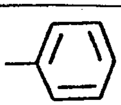 | H | $-(CH_2)_3CH_3$ | H | 261–262 |
| 57 | 3-COOH | 4-$OCOCH_3$ | $-(CH_2)_3CH_3$ | $-COCH_3$ | 164–166 |
| 58 | 3–$SCH_3$ | H | $-(CH_2)_3CH_3$ | $-COCH_3$ | 142–143 |
| 59 | 3-$SOCH_3$ | H | $-(CH_2)_3CH_3$ | $-COCH_3$ | 166–168 |

| Compound No. | Y | Z | $R_1$ | $R_2$ | m.p. °C. |
|---|---|---|---|---|---|
| 60 | H | H | $-(CH_2)_3CH_2OCOCH_3$ | H | 189–191 |
| 61 | H | H | $-CH_2C \equiv CH$ | $-CH_2C \equiv CH$ | 173–175 |
| 62 | 4-$CH_3$ | H | $-(CH_2)_3CH_2OCOCH_2CH_3$ | $-COCH_2CH_3$ | 100–102 |
| 63 | 4-Cl | H | $-(CH_2)_3CH_2OCOCH_2CH_3$ | $-COCH_2CH_3$ | 132–133.5 |
| 64 | 4-$CH_3$ | H | $-(CH_2)_3CH_2OCOCH_3$ | H | 208–210 |
| 65 | 4-Cl | H | $-(CH_2)_3CH_2OCOCH_3$ | H | 1/4 $H_2O$ (hydrate) 236–238 |
| 66 | 3-$CF_3$ | H | $-CH_2CH = CH_2$ | $-COCH_3$ | 105–107 |
| 67 | 3-Cl | 4-Cl | $-CH_2CH_2CH_2OH$ | H | 232–234 |
| 68 | 3-$OCH_3$ | H | $COCH_3$ | H | 265–267 |
| 69 | H | H | 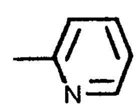 | H | |

| Compound No. | Y | Z | $R_1$ | $R_2$ | m.p. °C. |
|---|---|---|---|---|---|
| 70 | H | H | 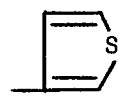 | H | |
| 71 | H | H | 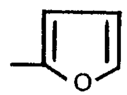 | H | |
| 72 | H | H | 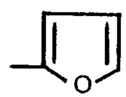 | H | |

The compounds of this invention are useful for treating chronic obstructive lung diseases such as asthma, bronchitis and the like because they have been shown by standard test procedures to inhibit the release of mediators such as SRS—A (slow reacting substance of anaphylaxis) and histamine and to antagonize the action of SRS—A on respiratory tissue. These chronic obstructive lung diseases can result from allergic reactions to have non-allergic causes. The compounds can thus be used to treat allergy caused diseases and their preferred use is for treating allergic chronic obstructive lung diseases. Chronic obstructive lung disease as used herein means disease conditions in which the passage of air through the lungs is obstructed or diminished such as is the case in asthma, bronchitis and the like.

The anti-allergic activity is identified by tests which measure a compound's inhibition of anaphylactic bronchospasm in sensitized guinea pigs having antigen induced SRS—A bronchoconstriction. The compounds are orally effective non-adrenergic, non-anticholinergic antianaphylactic agents. When administered orally they are active at dosages of from about 2 to 100 mg/kg of body weight; when administered parenterally, e.g. intravenously, the compounds are active at dosages of from about 1 to 10 mg/kg body weight.

In *in vitro* tests, the compounds antagonize contractions of lung parenchymal strips caused by leukotriene $C_4$. They are found to be active from these and other tests, as well as by comparison with compounds known to be effective for treating chronic obstructive lung diseases such as asthma or bronchitis. In the preferred anti-allergy use, the compounds of this invention are used to treat allergic patients by administering an anti-allergy effective amount thereof. The allergies treated can be, for example, asthma, as well as other chronic obstructive lung diseases.

Certain of the compounds described herein possess gastro-intestinal cytoprotectant effects.

The active compounds can be administered orally, topically, parenterally, or by oral or nasal inhalation. The preferred mode of administration is orally.

The amount and frequency of administration will be regulated according to the judgement of the attending clinician considering such factors as age, condition and size of the patient as well as severity of the disease being treated. A typical recommended dosage regimen is oral administration of from 200 to 1500 mg/day, preferably 500 to 800 mg/day, in two to four divided doses to achieve relief of the symptoms.

The compounds can be administered in conventional oral dosage forms such as capsules, tablets, pills, powders, suspensions or solutions prepared with conventional pharmaceutically acceptable excipients and additives, using conventional techniques. Parenteral preparations, i.e. sterile solutions or suspensions are also made by conventional means. Inhalation administration can be in the form of a nasal or oral spray. Insufflation is also contemplated. Topical dosage forms can be creams, ointments, lotions and the like. Other dosage forms which can be used are transdermal devices.

Pharmaceutical Dosage Form Examples

Example A
Tablets

| No. | Ingredient | mg/tablets | mg/tablet |
|---|---|---|---|
| 1. | Active compound | 100 | 500 |
| 2. | Lactose USP | 122 | 113 |
| 3. | Corn Starch, Food Grade, as a 10% paste in purified water | 30 | 40 |
| 4. | Corn Starch, Food Grade | 45 | 40 |
| 5. | Magnesium Stearate | 3 | 7 |
| | Total | 300 | 700 |

Method of Manufacture

Mix items Nos. 1 and 2 in a suitable mixer for 10—15 minutes. Granulate the mixture with Item No. 3. Mill the damp granules through a coarse screen (e.g., 1/4") if needed. Dry the damp granules. Screen the dried granules if needed and mix with Item No. 4 and mix for 10—15 minutes. Add Item No. 5 and mix for 1—3 minutes. Compress the mixture to appropriate size and weight on a suitable tablet machine.

Example B
Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade, | 40 | 70 |
| 4. | Magnesium Stearate NF | 4 | 7 |
| | Total | 250 | 700 |

Method of Manufacture

Mix Items Nos. 1, 2 and 3 in a suitable blender for 10—15 minutes. Add Item No. 4 and mix for 1—3 minutes. Fill the mixture into suitable two-piece hard gelatin capsules on a suitable encapsulating machine.

Example C
Parenteral

| Ingredient | Mg/vial | mg/vial |
|---|---|---|
| Active Compound Sterile Powder | 100 | 500 |

Method of manufacture

Add sterile water for injection or bacteriostatic water for injection for reconstitution.

Example D
Injectable

| Ingredient | mg/vial | mg/vial |
|---|---|---|
| Active compound | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s ad | 1.0 ml | 1.0 ml |

Method of Manufacture

1. Dissolve parabens in a portion (85% of the final volume) of the water for injection at 65—70°C.
2. Cool to 25—35°C. Charge and dissolve the sodium bisulfite, disodium edetate and sodium sulfate.
3. Charge and dissolve drug.
4. Bring the solution to final volume by added water for injection.
5. Filter the solution through 0.22 membrane and fill into appropriate containers.
6. Terminally sterilize the units by autoclaving.

**Claims for the Contracting States: BE CH DE FR GB IT LI NL SE**

1. Compounds of the general Formula I

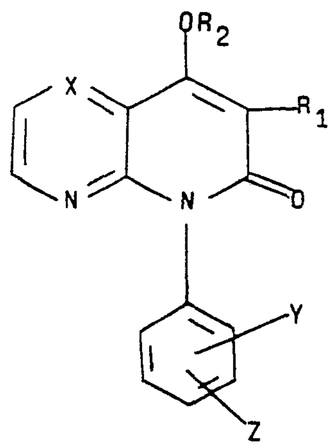

I

wherein

X is CH or N;

Y is hydrogen, hydroxy, benzyloxy, amino, sulfamyl, halogen, nitro, alkyl having from 1 to 6 carbon atoms, alkoxy having from 1 to 6 carbon atoms, carboxylic acyl having from 2 to 6 carbon atoms, alkyl-$S(O)_m$- having from 1 to 6 carbon atoms wherein m is 0, 1 or 2, trifluoromethyl, trifluoromethylthio, or COOA wherein A is hydrogen, alkyl having from 1 to 6 carbon atoms or a pharmaceutically acceptable cation derived from a metal or an amine;

Z is hydrogen, hydroxy, halogen, alkyl having from 1 to 6 carbon atoms, alkoxy having from 1 to 6 carbon atoms, hydroxyalkyl having from 1 to 6 carbon atoms or carboxylic acyloxy having from 2 to 6 carbon atoms;

$R_1$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 4- or 5-pyrimidyl, 2-, 3- or 4-pyridyl, 2- or 3-thienyl, 2- or 3-furanyl, carboxylic acyl having from 2 to 6 carbon atoms, or alkyl having from 1 to 10 carbon atoms which alkyl may be substituted with hydroxy, halogen, alkoxy having from 1 to 6 carbon atoms, carboxylic acyl having from 2 to 6 carbon atoms, phenyl, 2-, 3- or 4-pyridyl, 2-, 4- or 5-pyrimidyl, 2- or 3-thienyl, 2- or 3-furanyl, cycloalkyl having from 3 to 7 carbon atoms or carboxylic acyloxy having from 1 to 6 carbon atoms.

$R_2$ is hydrogen, carboxylic acyl having from 1 to 6 carbon atoms, alkyl having from 1 to 6 carbon atoms, hydroxyalkyl having 2 to 6 carbon atoms, dihydroxyalkyl having from 2 to 6 carbon atoms, alkenyl having from 2 to 6 carbon atoms, alkynyl having from 2 to 6 carbon atoms, $R_aR_bN(CH_2)_n$– wherein n is an integer of from 2 to 6, $R_a$ and $R_b$ are hydrogen, alkyl having from 1 to 6 carbon atoms or form, together with the nitrogen atom to which they are attached, a piperidine, morpholine, piperazine or pyrrolidine ring or $R_2$ represents a pharmaceutically acceptable cation derived from a metal or an amine, with the proviso that when X is CH, $R_2$ is hydrogen or carboxylic acyl, Y is hydrogen, alkyl, alkoxy, $CF_3$, $CH_3SO_2$, —$SCF_3$ or $NO_2$ *and* Z is hydrogen, halogen, alkyl or alkoxy, *then* $R_1$ is not unsubstituted alkyl; the acid addition salts and hydrates and solvates of such compounds.

2. Compounds according to Claim 1, wherein X is CH.

3. Compounds according to Claim 1 or 2, wherein Y is hydrogen, $C_1$ to $C_6$-alkyl, $C_1$ to $C_6$-alkoxy, $CF_3$, $SCF_3$ or $NO_2$, Z is hydrogen, halogen, $C_1$ to $C_6$-alkyl, or $C_1$ to $C_6$-alkoxy, $R_1$ is unsubstituted alkyl having from 1 to 10 carbon atoms and $R_2$ is a cation derived from a pharmaceutically acceptable metal or an amine.

4. Compounds according to Claim 2, wherein Y and Z are independently selected from hydrogen, $C_1$ to $C_6$-alkyl and $C_1$ to $C_6$-alkoxy, $R_1$ is alkenyl having 3 or 4 carbon atoms, ω-hydroxyalkyl having 2 to 4 carbon atoms, ω-carboxylicacyloxyalkyl having from 6 to 9 carbon atoms or *n*-alkyl having from 3 to 5 carbon atoms, and $R_2$ is a cation derived from a metal or an amine, —$CH_2CH_2OH$ or, provided $R_1$ is not n-alkyl, also hydrogen or acyl having from 1 to 6 carbon atoms.

5. Compounds according to Claim 4, wherein

Y is hydrogen, methylthio or methoxy,

Z is hydrogen or methyl,

$R_1$ is n-$C_4H_9$, —$(CH_2)_3CH_2OCOC_2H_5$, —$CH_2CH_2OH$, —$(CH_2)_2CH_2OH$ or —$CH_2$-$CH = CH_2$ and

$R_2$ is sodium cation or, provided $R_1$ is not n-$C_4H_9$, also hydrogen —$COCH_3$ or —$COC_2H_5$.

6. A compound according to Claim 3, being the sodium, potassium, calcium, ethanolamine, N-methylglucamine, diethanolamine, ethylenediamine, tris-hydroxy-ethyl-methylamine or lysine salt of 3-(n-butyl)-4-hydroxy-1-phenyl-1,8-naphythridine-2-(1*H*)-one.

7. A compound according to Claim 6, being the sodium salt of 3-(n-butyl)-4-hydroxy-1-phenyl-1,8-naphthyridine-2(1*H*)-one.

8. Compounds according to Claim 5, being
4-acetoxy-1-phenyl-3-(2-propenyl)-1,8-naphthyridin-2(*H*)-one;
3-(n-butyl)-4-(2-hydroxyethoxy)-1-phenyl-1,8-naphthyridin-2(1*H*)-one;
1-phenyl-4-propionyloxy-3-(4-propionyloxybutyl)-1,8-naphthyridin-2(1*H*)-one.

9. Compounds according to Claim 5 being
4-hydroxy-1-(3-methoxyphenyl)-3-(2-propenyl)-1,8-naphthyridin-2(1*H*)-one;
4-hydroxy-3-(2-hydroxyethyl)-1-phenyl-1,8-naphthyridin-2(1*H*)-one.

10. Process for the preparation of compounds of Formula I defined in Claim 1, caharacterized in that:
A: A compound of the general Formula II

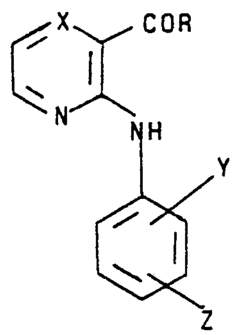

(II)

wherein X, Y and Z are as defined above and R represents any convenient leaving group, preferably an alkoxy group, is reacted with a compound of the general Formula III

$$R_1CH_2COR \quad (III)$$

wherein $R_1$ and R are as defined above; or

B: for the preparation of a compound of Formula I wherein $R_1$ represents an alkenyl group with 3 to 6 carbon atoms having the double bond in the 2,3-position, a compound of the general formula IV

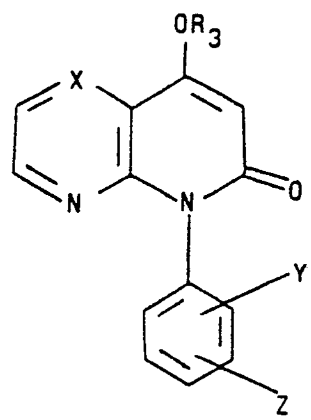

(IV)

wherein $R_3$ is an alkenyl group with 3 to 6 carbon atoms having the double bond in the 2,3-position and X, Y and Z are as defined, is subjected to a Claisen rearrangement, and that a compound obtained according to process A or B above wherein $R_2$ represents hydrogen, if desired, is subjected to introduction of a group $R_3$ different from hydrogen.

11. Pharmaceutical compositions comprising as an active ingredient a compound as defined in any one of claims 1 to 9.

12. Process for preparing pharmaceutical compositions, characterized in that a compound as defined in any one of claims 1 to 9, is brought into a form suitable for pharmaceutical administration.

**Claims for the Contracting State: AT**

1. Process for preparing the compounds of the general Formula I

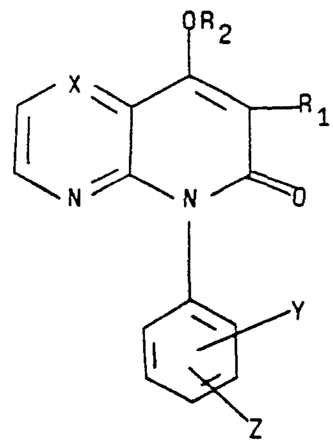

I

wherein

X is CH or N;

Y is hydrogen, hydroxy, benzyloxy, amino, sulfamyl, halogen, nitro, alkyl having from 1 to 6 carbon atoms, alkoxy having from 1 to 6 carbon atoms, carboxylic acyl having from 2 to 6 carbon atoms, alkyl-$S(O)_m$- having from 1 to 6 carbon atoms wherein m is 0, 1 or 2, trifluoromethyl, trifluoromethylthio, or COOA wherein A is hydrogen, alkyl having from 1 to 6 carbon atoms or a pharmaceutically acceptable cation derived from a metal or an amine;

Z is hydrogen, hydroxy, halogen, alkyl having from 1 to 6 carbon atoms, alkoxy having from 1 to 6 carbon atoms, hydroxyalkyl having 1 to 6 carbon atoms or carboxylic acyloxy having from 2 to 6 carbon atoms;

$R_1$ is alkenyl having from 2 to 10 carbon atoms, alkynyl having from 2 to 10 carbon atoms, cycloalkyl having from 3 to 7 carbon atoms, cycloalkenyl having from 5 to 8 carbon atoms, 2-, 4- or 5-pyrimidyl, 2-, 3- or 4-pyridyl, 2- or 3-thienyl, 2- or 3-furanyl, carboxylic acyl having from 2 to 6 carbon atoms, or alkyl having from 1 to 10 carbon atoms which alkyl may be substituted with hydroxy, halogen, alkoxy having from 1 to 6 carbon atoms, carboxylic acyl having from 2 to 6 carbon atoms, phenyl, 2-, 3- or 4-pyridyl, 2-, 4- or 5-pyrimidyl, 2- or 3-thienyl, 2- or 3-furanyl, cycloalkyl having from 3 to 7 carbon atoms or carboxylic acyloxy having from 1 to 6 carbon atoms.

$R_2$ is hydrogen, carboxylic acyl having from 1 to 6 carbon atoms, alkyl having from 1 to 6 carbon atoms, hydroxyalkyl having from 2 to 6 carbon atoms, dihydroxyalkyl having from 2 to 6 carbon atoms, alkenyl having from 2 to 6 carbon atoms, alkynyl having from 2 to 6 carbon atoms, $R_aR_bN(CH_2)_n$- wherein n is an integer of from 2 to 6, $R_a$ and $R_b$ are hydrogen, alkyl having from 1 to 6 carbon atoms or form, together with the nitrogen atom to which they are attached, a piperidine, morpholine, piperazine or pyrrolidine ring or $R_2$ represents a pharmaceutically acceptable cation derived from a metal or an amine; with the proviso that when X is CH, $R_2$ is hydrogen or carboxylic acyl, Y is hydrogen, alkyl, alkoxy, $CF_3$, $CH_3SO_2$, $—SCF_3$ or $NO_2$ *and* Z is hydrogen, halogen, alkyl or alkoxy, *then* $R_1$ is not unsubstituted alkyl; the acid addition salts and hydrates and solvates of such compounds, characterized in that:

A: A compound of the general Formula II

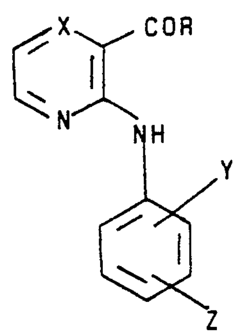

(II)

wherein X, Y and Z are as defined above and R represents any convenient leaving group, preferably an alkoxy group, is reacted with a compound of the general Formula III

$$R_1CH_2COR \qquad (III)$$

wherein $R_1$ and R are as defined above; or

B: for the preparation of a compound of Formula I wherein $R_1$ represents an alkenyl group with 3 to 6 carbon atoms having the double bond in the 2,3-position, a compound of the general formula IV

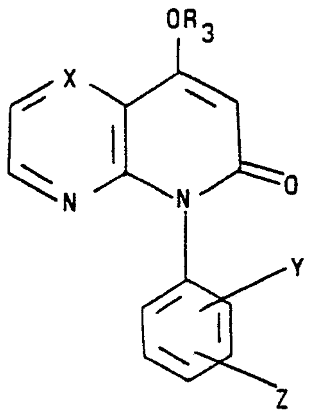

(IV)

wherein $R_3$ is an alkenyl group with 3 to 6 carbon atoms having the double bond in the 2,3-position and X, Y and Z are as above defined, is subjected to a Claisen rearrangement, and that a compound obtained according to process A or B above wherein $R_2$ represents hydrogen, if desired, is subjected to introduction of a group $R_2$ different from hydrogen.

2. Process according to Claim 1B, characterized in that the Claisen rearrangement is carried out in the presence of an acid anhydride whereby a compound of Formula I wherein $R_1$ is alkenyl group with 3 to 6 carbon atoms having the double bond in the 2,3-position and $R_2$ is an acyl group is obtained.

3. Process according to claim 1A, characterized in that the sodium, potassium, calcium, ethanolamine, N-methylglucamine, diethanolamine, ethylenediamine, tris-hydroxyethyl-methylamine or lysine salt of 3-(n-butyl)-4-hydroxy-1-phenyl-1,8-naphthyridine-2-(1*H*)-one is prepared.

4. Process according to claim 1, characterized in that a compound of Formula I wherein

Y is hydrogen, methylthio or methoxy;

Z is hydrogen or methyl,

$R_1$ is n-$C_4H_9$, $—(CH_2)_3CH_2OCOC_2H_5$, $—CH_2CH_2OH$, $—(CH_2)_2CH_2OH$ or $—CH_2\text{-}CH = CH_2$ and

$R_2$ is sodium cation or, provided $R_1$ is not n-$C_4H_9$, also hydrogen $—COCH_3$ or $—COC_2H_5$ is prepared.

5. Process according to claim 4, characterized in that 4-acetoxy-1-phenyl-3-(2-propenyl)-1,8-naphthyridin-2(1*H*)-one is prepared.

6. Process according to claim 4, characterized in that 4-hydroxy-1-(3-methoxyphenyl)-3-(2-propenyl)-1,8-naphthyridine-2(1*H*)-one or the sodium salt thereof is prepared.

7. Process for preparing pharmaceutical compositions, characterized in that a compound as defined in any one of claims 1 to 6 is brought into a form suitable for pharmaceutical administration.

**Patentansprüche für die Vertragsstaaten: BE CH DE FR GB IT LI NL SE**

1. Verbindungen der allgemeinen Formel I

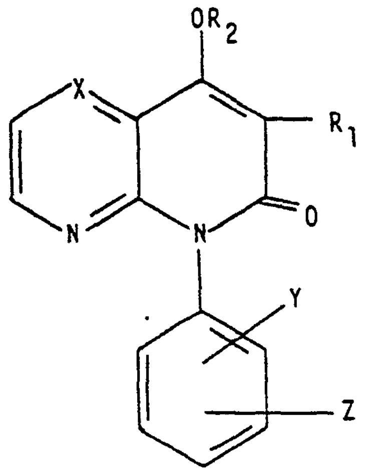

I

worin

X CH oder N ist;

Y Wasserstoff, Hydroxy, Benzyloxy, Amino, Sulfamyl, Halogen, Nitro, Alkyl mit 1 bis 6 Kohlenstoffatomen, Alkoxy mit 1 bis 6 Kohlenstoffatomen, Carbonsäureacyl mit 2 bis 6 Kohlenstoffatomen, Alkyl-$S(O)_m$— mit 1 bis 6 Kohlenstoffatomen, worin m 0, 1 oder 2 ist, Trifluormethyl, Trifluormethylthio oder COOA bedeutet, worin A Wasserstoff, Alkyl mit 1 bis 6 Kohlenstoffatomen oder ein pharmazeutisch annehmbares von einem Metall oder einem Amin abgeleitetes Kation ist;

Z Wasserstoff, Hydroxy, Halogen, Alkyl mit 1 bis 6 Kohlenstoffatom, Alkoxy mit 1 bis 6 Kohlenstoffatomen, Hydroxyalkyl mit 1 bis 6 Kohlenstoffatomen oder Carbonsäureacyloxy mit 2 bis 6 Kohlenstoffatomen ist; $R_1$ Alkenyl mit 2 bis 10 Kohlenstoffatomen, Alkinyl mit 2 bis 10 Kohlenstoffatomen, Cycloalkyl mit 3 bis 7 Kohlenstoffatomen, Cycloalkenyl mit 56 bis 8 Kohlenstoffatomen, 2-, 4- oder 5-Pyrimidyl, 2-, 3- oder 4-Pyridyl, 2- oder 3-Thienyl, 2- oder 3-Furanyl, Carbonsäureacyl mit 2 bis 6 Kohlenstoffatomen oder Alkyl mit 1 bis 10 Kohlenstoffatomen ist, wobei das Alkyl durch Hydroxy, Halogen, Alkoxy mit 1 bis 6 Kohlenstoffatomen, Carbonsäureacyl mit 2 bis 6 Kohlenstoffatomen, Phenyl, 2-, 3- oder 4-Pyridyl, 2-, 4- oder 5-Pyrimidyl, 2- oder 3-Thienyl, 2- oder 3-Furanyl, Cycloalkyl mit 3 bis 7 Kohlenstoffatomen oder Carbonsäureacyloxy mit 1 bis 6 Kohlenstoffatomen substituiert sein kann;

$R_2$ Wasserstoff, Carbonsäureacyl mit 1 bis 6 Kohlenstoffatomen, Alkyl mit 1 bis 6 Kohlenstoffatomen, Hydroxyalkyl mit 2 bis 6 Kohlenstoffatomen, Dihydroxyalkyl mit 2 bis 6 Kohlenstoffatomen, Alkenyl mit 2 bis 6 Kohlenstoffatomen, Alkinyl mit 2 bis 6 Kohlenstoffatomen, $R_aR_bN(CH_2)_n$—, worin n eine ganze Zahl von 2 bis 6 ist, $R_a$ und $R_b$ Wasserstoff, Alkyl mit 1 bis 6 Kohlenstoffatomen sind oder zusammen mit dem Stickstoffatom, and das sie gebunden sind, einen Piperidin-, Morpholin-, Piperazin- oder Pyrrolidinring bilden, bedeutet, oder $R_2$ ein pharmazeutisch annehmbares von einem Metall oder einem Amin abgeleitetes Kation ist;

mit der Maßgabe, daß, wenn X CH,$R_2$ Wasserstoff oder Carbonsäureacyl, Y Wasserstoff, Alkyl, Alkoxy, $CF_3$, $CH_3SO_2$, —$SCF_3$ oder $NO_2$ und Z Wasserstoff, Halogen, Alkyl oder Alkoxy ist, $R_1$ nicht unsubstituiertes Alkyl bedeutet;

die Säureadditionssalze und Hydrate und Solvate solcher Verbindungen.

2. Verbindungen nach Anspruch 1, worin X CH ist.

3. Verbindungen nach Anspruch 1 oder 2, worin Y Wasserstoff, $C_1$- bis $C_6$-Alkyl, $C_1$- bis $C_6$-Alkoxy, $CF_3$, $SCF_3$ oder $NO_2$ ist, Z Wasserstoff, Halogen, $C_1$- bis $C_6$-Alkyl oder $C_1$- bis $C_6$-Alkoxy ist, $R_1$ unsubstituiertes Alkyl mit 1 bis 10 Kohlenstoffatomen ist, und $R_2$ ein von einem pharmazeutische annehmbaren Metall oder einem Amin abgeleitetes Kation ist.

4. Verbindungen nach Anspruch 2, worin Y und Z unabhängig voneinander aus Wasserstoff, $C_1$- bis $C_6$-Alkyl und $C_1$- bis $C_6$-Alkoxy ausgewählt sind, $R_1$ Alkenyl mit 3 oder 4 Kohlenstoffatomen, ω-Hydroxyalkyl mit 2 bis 4 Kohlenstoffatomen, ω-Carbonsäureacyloxyalkyl mit 6 bis 9 Kohlenstoffatomen oder n-Alkyl mit 3 bis 5 Kohlenstoffatomen ist, und $R_2$ ein von einem Metall oder einem Amin abgeleitetes Kation, —$CH_2CH_2OH$ oder, vorausgesetzt, $R_1$ ist nicht n-Alkyl, auch Wasserstoff oder Acyl mit 1 bis 6 Kohlenstoffatomen ist.

5. Verbindungen nach Anspruch 4, worin Y Wasserstoff, Methylthio oder Methoxy ist, Z Wasserstoff oder Methyl ist, $R_1$ n-$C_4H_9$, $—(CH_2)_3CH_2OCOC_2H_5$, $—CH_2CH_2OH$, $—(CH_2)_2CH_2OH$ oder $—CH_2—CH=CH_2$ ist und $R_2$ Natriumkation oder, vorausgesetzt $R_1$ ist nicht n-$C_4H_9$, auch Wasserstoff, $—COCH_3$ oder $—COC_2H_5$ ist.

6. Verbindungen nach Anspruch 3, welche das Natrium-, Kalium-, Kalzium-, Ethanolamin-, N-Methylglucamin-, Diethanolamin-, Ethylendiamin-, Tris-hydroxyethyl-methylamin- oder Lysin-salz von 3-(n-Butyl)-4-hydroxy-1-phenyl-1,8-naphthyridin-2(1H)-on ist.

7. Verbindungen nach Anspruch 6, welche das Natriumsalz von 3-(n-Butyl)-4-hydroxy-1-phenyl-1,8-naphthyridin-2-(1H)-on ist.

8. Verbindungen nach Anspruch 5, welche
4-Acetoxy-1-phenyl-3-(2-propenyl)-1,8-naphthyridin-2-(1H)-on;
3-(n-Butyl)-4-(2-hydroxyethoxy)-1-phenyl-1,8-naphthyridin-2(1H)- on; bzw.
1-Phenyl-4-propionyloxy-3-(4-propionyloxybutyl)-1,8-naphthyridin-2(1H)-on sind.

9. Verbindungen nach Anspruch 5, welche
4-Hydroxy-1-(3-methoxyphenyl)-3-(2-propenyl)-1,8-naphthyridin-2(1H)-on; bzw.
4-Hydroxy-3-(2-hydroxyethyl)-1-phenyl-1,8-naphthyridin-2(1H)-on sind.

10. Verfahren zur Herstellung von Verbindungen der in Anspruch 1 definierten Formel I, gekennzeichnet, daß

A: eine Verbindung der allgemeinen Formel II

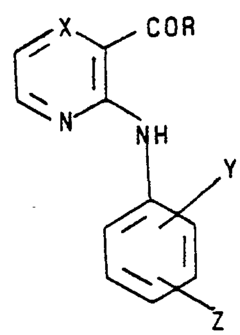

(II)

worin X, Y und Z die oben angegebenen Bedeutungen haben, und R irgendeine entsprechende Abgangsgruppe, vorzugsweise eine Alkoxygruppe, darstellt, mit einer Verbindung der allgemeinen Formel III

$$R_1CH_2COR \qquad (III)$$

worin $R_1$ und R obige Bedeutungen haben, umgesetzt wird; oder

B: zur Herstellung einer Verbindung der Formel I, worin $R_1$ ein Alkenylgruppe mit 3 bis 6 Kohlenstoffatomen und der Doppelbindung in der 2,3-Stellung darstellt, eine Verbindung der allgemeinen Formel IV.

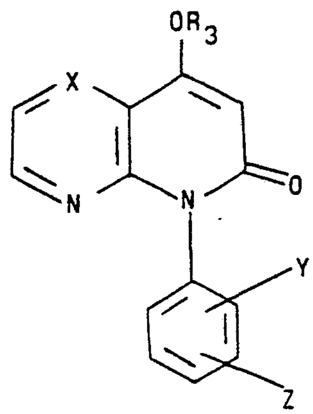

(IV)

worin $R_3$ eine Alkenylgruppe mit 3 bis 6 Kohlenstoffatomen und der Deoppelbindung in der 2,3-Stellung ist und X, Y und Z obige Bedeutung haben, einer Claisen-Umlagerung unterworfen wird, und daß eine gemäß dem obigen Verfahren A oder B erhaltene Verbindung, worin $R_2$ Wasserstoff darstellt, falls gewünscht, der Einführung einer von Wasserstoff verschiedenen Gruppe $R_2$ unterworfen wird.

11. Pharmazeutische Zusammensetzungen, welche als einen Wirkstoff eine in einem der Ansprüche 1 bis 9 definierte Verbindung enthalten.

12. Verfahren zur Herstellung pharmazeutischer Zusammensetzungen, dadurch gekennzeichnet, daß eine in einem der Ansprüche 1 bis 9 definierte Verbindung in eine für die pharmaceutische Verabreichung geeignete Form gebracht wird.

**Patentansprüche für deu Vertragstaat: AT**

1. Verfahren zur Herstellung der Verbindungen der allgemeinen Formel I

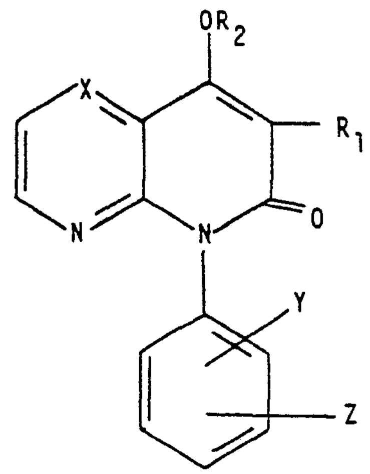

I

worin

X CH oder N ist;

Y Wasserstoff, Hydroxy, Benzyloxy, Amino, Sulfamyl, Halogen, Nitro, Alkyl mit 1 bis 6 Kohlenstoffatomen, Alkoxy mit 1 bis 6 Kohlenstoffatomen, Carbonsäureacyl mit 2 bis 6 Kohlenstoffatomen, Alkyl-$S(O)_m$— mit 1 bis 6 Kohlenstoffatomen, worin m 0, 1 oder 2 ist, Trifluormethyl, Trifluormethylthio oder COOA bedeutet, worin A Wasserstoff, Alkyl mit 1 bis 6 Kohlenstoffatomen oder ein pharmazeutisch annehmbares von einem Metall oder einem Amin abgeleitetes Kation ist;

Z Wasserstoff, Hydroxy, Halogen, Alkyl mit 1 bis 6 Kohlenstoffatom, Alkoxy mit 1 bis 6 Kohlenstoffatomen, Hydroxyalkyl mit 1 bis 6 Kohlenstoffatomen oder Carbonsäureacyloxy mit 2 bis 6 Kohlenstoffatomen ist; $R_1$ Alkenyl mit 2 bis 10 Kohlenstoffatomen, Alkinyl mit 2 bis 10 Kohlenstoffatomen, Cycloalkyl mit 3 bis 7 Kohlenstoffatomen, Cycloalkenyl mit 5 bis 8 Kohlenstoffatomen, 2-, 4- oder 5-Pyrimidyl, 2-, 3- oder 4-Pyridyl, 2- oder 3-Thienyl, 2- oder 3-Furanyl, Carbonsäureacyl mit 2 bis 6 Kohlenstoffatomen oder Alkyl mit 1 bis 10 Kohlenstoffatomen ist, wobei das Alkyl durch Hydroxy, Halogen, Alkoxy mit 1 bis 6 Kohlenstoffatomen, Carbonsäureacyl mit 2 bis 6 Kohlenstoffatomen, Phenyl, 2-, 3- oder 4-Pyridyl, 2-, 4- oder 5-Pyrimidyl, 2- oder 3-Thienyl, 2- oder 3-Furanyl, Cycloalkyl mit 3 bis 7 Kohlenstoffatomen oder Carbonsäureacyloxy mit 1 bis 6 Kohlenstoffatomen substituiert sein kann;

$R_2$ Wasserstoff, Carbonsäureacyl mit 1 bis 6 Kohlenstoffatomen, Alkyl mit 1 bis 6 Kohlenstoffatomen, Hydroxyalkyl mit 2 bis 6 Kohlenstoffatomen, Dihydroxyalkyl mit 2 bis 6 Kohlenstoffatomen, Alkenyl mit 2 bis 6 Kohlenstoffatomen, Alkinyl mit 2 bis 6 Kohlenstoffatomen, $R_aR_bN(CH_2)_n$—, worin n eine ganze Zahl von 2 bis 6 ist, $R_a$ und $R_b$ Wasserstoff, Alkyl mit 1 bis 6 Kohlenstoffatomen sind oder zusammen mit dem Stickstoffatom, and das sie gebunden sind, einen Piperidin-, Morpholin-, Piperazin- oder Pyrrolidinring bilden, bedeutet, oder $R_2$ ein pharmazeutisch annehmbares von einem Metall oder einem Amin abgeleitetes Kation ist;

mit der Maßgabe, daß, wenn X CH $R_2$ Wasserstoff oder Carbonsäureacyl, Y Wasserstoff, Alkyl, Alkoxy, $CF_3$, $CH_3SO_2$, —$SCF_3$ oder $NO_2$ und Z Wasserstoff, Halogen, Alkyl oder Alkoxy ist, $R_1$ nicht unsubstituiertes Alkyl bedeutet;

der Säureadditionssalze und Hydrate und Solvate solcher Verbindungen, dadurch gekennzeichnet, daß

A: eine Verbindung der allgemeinen Formel II

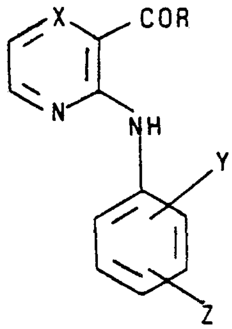

(II)

worin X, Y und Z die oben angegebenen Bedeutungen haben, und R irgendeine entsprechende Abgangsgruppe, vorzugsweise eine Alkoxygruppe, darstellt, mit einer Verbindung der allgemeinen Formel III

$$R_1CH_2COR \qquad (III)$$

worin $R_1$ und R obige Bedeutungen haben, umgesetzt wird; oder

B: zur Herstellung einer Verbindung der Formel I, worin $R_1$ ein Alkenylgruppe mit 3 bis 6 Kohlenstoffatomen und der Doppelbindung in der 2,3-Stellung darstellt, eine Verbindung der allgemeinen Formel IV.

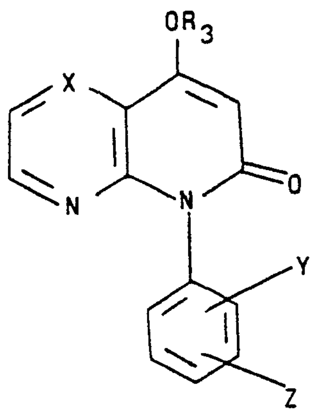

(IV)

worin $R_3$ eine Alkenylgruppe mit 3 bis 6 Kohlenstoffatomen und der Deoppelbindung in der 2,3-Stellung ist und X, Y und Z obige Bedeutung haben, einer Claisen-Umlagerung unterworfen wird, und daß eine gemäß dem obigen Verfahren A oder B erhaltene Verbindung, worin $R_2$ Wasserstoff darstellt, falls erwünscht, der Einführung einer von Wasserstoff verschiedenen Gruppe $R_2$ unterworfen wird.

2. Verfahren nach Anspruch 1B, dadurch gekennzeichnet, daß die Claisen-Umlagerung in Gegenwart eines Säureanhydrids ausgeführt wird, wodurch eine Verbindung der Formel I, worin $R_1$ eine Alkenylgruppe mit 3 bis 6 Kohlenstoffatomen und der Doppelbindung in der 2,3-Stellung und $R_2$ eine Acylgruppe ist, erhalten wird.

3. Verfahren nach Anspurch 1A dadurch gekennzeichnet, daß das Natrium-, Kalium-, Kalzium-, Ethanolamin-, n-Methylglucamin-, Diethanolamin-, Ethylendiamin-, Tris-hydroxyethyl-methylamin- oder Lysinsalz von 3-(n-Butyl)-4-hyroxy-1-phenyl-1,8-naphthyridin-2(1H)-on herstellt wird.

4. Verfahren nach Anspurch 1, dadurch gekennzeichnet, daß eine Verbindung der Formel I, worin Y Wasserstoff, Methylthio oder Methoxy ist, Z Wasserstoff oder Methyl ist, $R_1$ n-$C_4H_9$, $-(CH_2)_3CH_2OCOC_2H_5$, $-CH_2CH_2OH$, $-(CH_2)_2CH_2OH$ oder $-CH_2-CH=CH_2$ ist und $R_2$ Natriumkation oder, vorausgesetzt $R_1$ ist nicht n-$C_4H_9$, auch Wasserstoff, $-COCH_3$ oder $-COC_2H_5$ ist, hergestellt wird.

5. Verfahren nach Anspurch 4, dadurch gekennzeichnet, daß 4-Acetoxy-1-phenyl-3-(2-propenyl)-1,8-naphthyridin-2(1H)-on hergestellt wird.

6. Verfahren nach Anspurch 4, dadurch gekennzeichnet, daß 4-Hydroxy-1-(3-methoxyphenyl)-3-(2-propenyl)-1,8-naphthyridin-2(1H)-on oder das Natriumsalz davon hergestellt wird.

7. Verfahren zur Herstellung pharmazeutischer Zusammensetzungen, dadurch gekennzeichnet, daß eine in einem der Ansprüche 1 bis 6 definierte Verbindung in eine für die pharmazeutische Verabreichung geeignete Form begracht wird.

**Revendications pour les Etats contractants: BE CH DE FR GB IT LI NL SE**

1. Composés de la formule générale I

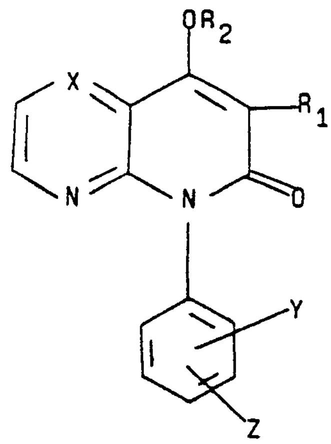

I

dand lauqelle

X signifie CH ou N;

Y signifie hydrogène, hydroxy, benzyloxy, amino, sulfamyle, halogène, nitro, alcoyle ayant de 1 à 6 atomes de carbone, alcoxy ayant de 1 à 6 atomes de carbone, acyl carboxylique ayant de 2 à 6 atomes de carbone, alcoyl-$S(O)_m$— ayant de 1 à 6 atomes de carbone où m est 0, 1 ou 2, trifluorométhyle, trifluorométhylthio, ou COOA où A signifie hydrogène, alcoyle ayant de 1 à 6 atomes de carbone ou un cation pharmaceutiquement acceptable dérivé d'un métal ou d'une amine;

Z signifie hydrogène, hydroxy, halogène, alcoyle ayant de 1 à 6 atomes de carbone, alcoxy ayant de 1 à 6 atomes de carbone, hydroxyalcoyle ayant de 1 à 6 atomes de carbone ou acyloxy carboxylique ayant de 2 à 6 atomes de carbone;

$R_1$ signifie alcényle ayant de 2 à 10 atomes de carbone, alcynyle ayant de 2 à 10 atomes de carbone, cycloalcoyle ayant de 3 à 7 atomes de carbone, cycloalcényle ayant de 5 à 8 atomes de carbone, 2-, 4- ou 5-pyrimidyle, 2-, 3- ou 4-pyridyle, 2- ou 3-thiényle, 2- ou 3-furanyle, acyle carboxylique ayant de 2 à 6 atomes de carbone, ou alcoyle ayant de 1 à 10 atomes de carbone, lequel alcoyle peut être substitué par hydroxy, halogène, alcoxy ayant de 1 à 6 atomes de carbone, acyle carboxylique ayant de 2 à 6 atomes de carbone, phényle, 2-, 3- ou 4-pyridyle, 2-, 4- ou 5-pyrimidyle, 2- ou 3-thiényle, 2- ou 3-furanyle, cycloalcoyle ayant de 3 à 7 atomes de carbone ou acyloxy carboxylique ayant de 1 à 6 atomes de carbone;

$R_2$ signifie hydrogène acyle carboxylique ayant de 1 à 6 atomes de carbone, alcoyle ayant de 1 à 6 atomes de carbone, hydroxyalcoyle ayant de 2 à 6 atomes de carbone, dihydroxyalcoyle ayant de 2 à 6 atomes de carbone, alcényle ayant de 2 à 6 atomes de carbone, alcynyle ayant de 2 à 6 atomes de carbone $R_aR_bN(CH_2)_n$— où n est un nombre entier de 2 à 6, $R_a$ et $R_b$ signifient hydrogène, alcoyle ayant de 1 à 6 atomes de carbone, ou forment avec l'atome d'azote auquel ils sont liés, un noyau de pipéridine, morpholine, pipérazine ou pyrrolidine, ou $R_2$ représente un cation pharamceutiquement acceptable dérivé d'un métal ou d'une amine;

à la condition que: quand X signifie CH, $R_2$ signifie hydrogène ou acyle carboxylique, Y signifie hydrogène, alcoyle, alcoxy, $CF_3$, $CH_3SO_2$, —$SCF_3$ ou $NO_2$ *et* Z signifie hydrogène, halogène, alcoyle ou alcoxy, *alors* $R_1$ n'est pas un alcoyle non substitué;

les sels d'addition acides et hydrates et solvates de tels composés.

2. Composés selon la revendication 1, dans lesquels X est CH.

3. Composés selon la revendication 1 ou 2, dans lesquels Y signifie hydrogène, alcoyle en $C_1$ à $C_6$, alcoxy en $C_1$ à $C_6$, $CF_3$, $SCF_3$ ou $NO_2$, Z signifie hydrogène, alcoyle en $C_1$ à $C_6$, ou alcoxy en $C_1$ à $C_6$, $R_1$ signifie alcoyle non substitué ayant de 1 à 10 atomes de carbone et $R_2$ est un cation dérivé d'un métal ou d'une amine pharmaceutiquement acceptable.

4. Composés selon la revendication 2, dans lesquels Y et Z sont choisis indépendamment dans le groupe comprenant: hydrogène, alcoyle en $C_1$ à $C_6$ et alcoxy en $C_1$ à $C_6$, $R_1$ signifie alcényle à 3 ou 4 atomes de carbone, ω-hydroxyalcoyle ayant 2 à 4 atomes de carbone, ω-acyloxyalcoyle-carboxylique ayant de 6 à 9 atomes de carbone ou *n*-alcoyle ayant de 3 à 5 atomes de carbone, et $R_2$ signfie un cation dérivé d'un métal ou d'une amine, —$CH_2CH_2OH$ ou, à condition que $R_1$ ne soit pas n-alcoyle, également hydrogène ou acyle ayant de 1 à 6 atomes de carbone.

5. Composés selon la revendication 4, dans lesquels Y signifie hydrogène, méthylthio ou méthoxy, Z signifie hydrogène ou méthyle, $R_1$ signifie n-$C_4H_9$, —$(CH_2)_3CH_2OCOC_2H_5$, —$CH_2CH_2OH$, —$(CH_2)_2CH_2OH$ ou

—$CH_2$—CH=$CH_2$ et $R_2$ est un cation de sodium ou, à condition que $R_1$ ne soit pas n-$C_4H_9$, également hydrogène, —$COCH_3$ ou —$COC_2H_5$.

6. Un composés selon la revendication 3, consistant en le sel de sodium, potassium, calcium, éthanolamine, N-méthylglucamine, diéthanolamine, éthylènediamine, trishydroxyéthyl-méthylamine ou lysine de 3-(n-butyl)-4-hydroxy-1-phényl-1,8-naphtyridine-2(1*H*)-one.

7. Un composé selon la revendication 6, consistant en le sel de sodium de 3-(n-butyl)-4-hydroxy-1-phényl-1,8-naphtyridine-2(*H*)-one.

8. Composés selon la revendication 5, consistant en
4-acétoxy-1-phényl-3-(2-propényl)-1,8-naphtyridine-2(1*H*)-one;
3-(n-butyl)-4-(2-hydroxyéthoxy)-1-phényl-1,8-naphtyridine-2(1*H*)-one.
1-phényl-4-propionyloxy-3-(4-propionyloxybutyl)-1,8-naphtyridine-2(1*H*)-one.

9. Composés selon la revendication 5 étant le 4-hydroxy-1-(3-méthoxyphényl)-3-(2-propényl)-1,8-naphtyridine-2(1*H*)-one;
4-hydroxy-3-(2-hydroxyéthyl)-1-phényl-1,8-naphtyridine-2(1*H*)-one.

10. Procédé de préparation de composés de la formule I défins dans la revendiction 1, caractérisé en ce que:

A: On fait réagir un composé de la formule générale II

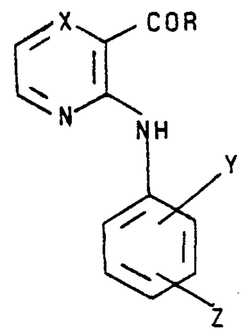

(II)

dans laquelle X, Y et Z ont les significations définies ci-dessus et R représente tout groupe partant convenable, de préférence un groupe alcoxy, avec un composé de la formule générale III

$$R_1CH_2COR \qquad (III)$$

dans laquelle $R_1$ et $R_3$ ont les significations définies ci-dessus- ou

B: pour la préparation d'un composé de la formule I dans laquelle $R_1$ représente un groupe alcényle avec 3 à 6 atomes de carbone ayatn une double liaison en position 2, 3, on soumet à un réarrangement de Claisen un composé de la fomule générale IV

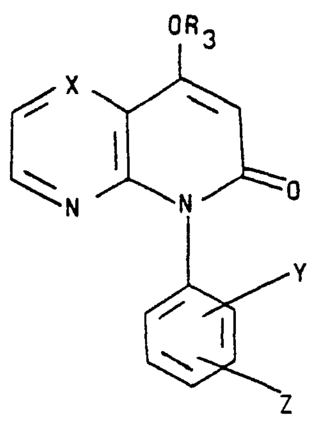

(IV)

dans laquelle $R_3$ est un groupe alcényle avec 3 à 6 atomes de carbone ayant une double liaison en position 2, 3 et X, Y et Z ont les significations définies ci-dessus, et en ce que un composé obtenu conformément au processus A ou B ci-dessus, dand laquel $R_2$ représente l'hydrogène, si on le désire, est soumis à l'introduction d'un groupe $R_2$ différent de l'hydrogène.

11. Compositions pharmaceutiques comprenant en tant qu'ingrédient actif un composé tel que défini dans l'une quelconque des revendications 1 à 9.

12. Procédé de préparation de compositions pharmaceutiques, caractérisé en ce qu'un composé comme défini dans l'un quelconque des revendications 1 à 9 est mis en une forme convenant pour son administration en tant que produit pharmaceutique.

**Revendications pour l'Etat contractants: AT**

1. Procédé de préparation de composés de la formule générale I

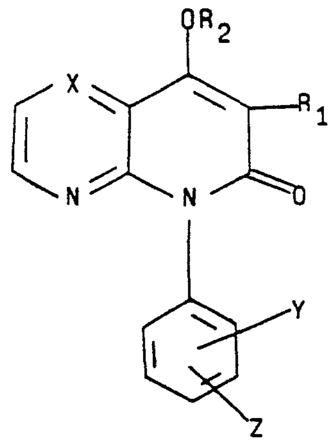

I

dans laquelle

X est CH ou N;

Y signifie hydrogène, hydroxy, benzyloxy, amino, sulfamyle, halogène, nitro, alcoyle ayant de 1 à 6 atomes de carbone, alcoxy ayant de 1 à 6 atomes de carbone, acyle carboxylique ayant de 2 à 6 atomes de carbone, alcoyle-$S(O)_m$— ayant de 1 à 6 atomes de carbone où m est 0, 1 ou 2, trifluorométhyle, trifluorométhylthio, ou COOA où A signifie hydrogène, alcoyle ayant de 1 à 6 atomes de carbone ou un cation pharmaceutiquement acceptable dérivé d'un métal ou d'une amine;

Z signifie hydrogène, hydroxy, halogène, alcoyle ayant de 1 à 6 atomes de carbone, alcoxy ayant de 1 à 6 atomes de carbone, hydroxyalcoyle ayant de 1 à 6 atomes de carbone ou acyloxy carboxylique ayant de 2 à 6 atomes de carbone;

$R_1$ signifie alcényle ayant de 2 à 10 atomes de carbone, alcynyle ayant de 2 à 10 atomes de carbone, cycloalcoyle ayant de 3 à 7 atomes de carbone, cycloalcényle ayant de 5 à 8 atomes de carbone, 2-, 4- ou 5-pyrimidyle, 2-, 3- ou 4-pyridyle, 2- ou 3-thiényle, 2- ou 3-furanyle, acyle carboxylique ayant de 2 à 6 atomes de carbone, ou alcoyle ayant de 1 à 10 atomes de carbone, lequel alcoyle peut être substitué par hydroxy, halogène, alcoxy ayant de 1 à 6 atomes de carbone, acyle carboxylique ayant de 2 à 6 atomes de carbone, phényle, 2-, 3- ou 4-pyridyle, 2-, 4- ou 5-pyrimidyle, 2- ou 3-thiényle, 2- ou 3-furanyle, cycloalcoyle ayant de 3 à 7 atomes de carbone ou acyloxy carboxylique ayant de 1 à 6 atomes de carbone;

$R_2$ signifie hydrogène acyle carboxylique ayant de 1 à 6 atomes de carbone, alcoyle ayant de 1 à 6 atomes de carbone, hydroxyalcoyle ayant de 2 à 6 atomes de carbone, dihydroxyalcoyle ayant de 2 à 6 atomes de carbone, alcényle ayant de 2 à 6 atomes de carbone, alcynyle ayant de 2 à 6 atomes de carbone $R_aR_bN(CH_2)_n$— où n est un nombre entier de 2 à 6, $R_a$ et $R_b$ signifient hydrogène, alcoyle ayant de 1 à 6 atomes de carbone, ou forment avec l'atome d'azote auquel il est lié, un noyau de pipéridine, morpholine, pipérazine ou pyrrolidine, ou $R_2$ représente un cation pharamceutiquement acceptable dérivé d'un métal ou d'une amine;

à la condition que: quand X signifie CH, $R_2$ signifie hydrogène ou acyle carboxylique, Y signifie hydrogène, alcoyle, alcoxy, $CF_3$, $CH_3SO_2$, $—SCF_3$ ou $NO_2$ *et* Z signifie hydrogène, halogène, alcoyle ou alcoxy, *alors* $R_1$ n'est pas un alcoyle non substitué;

les sels acide d'addition acides et hydrates et solvates de tels composés, caractérisé en ce que:

A: On fait réagir un composé de la formule générale II

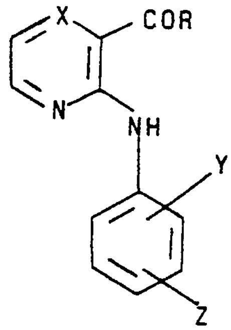

(II)

dans laquelle X, Y et Z ont les significations définies ci-dessus et R représente tout groupe partant convenable, de préférence un groupe alcoxy, avec un composé de la formule générale III

$$R_1CH_2COR \qquad (III)$$

dans laquelle $R_1$ et $R_3$ ont les significations définies ci-dessus- ou

B: pour la préparation d'un composé de la formule I dans laquelle $R_1$ représente un groupe alcényle avec 3 à 6 atomes de carbone ayant une double liaison en position 2, 3, on soumet à un réarrangement de Claisen un composé de la formule générale IV

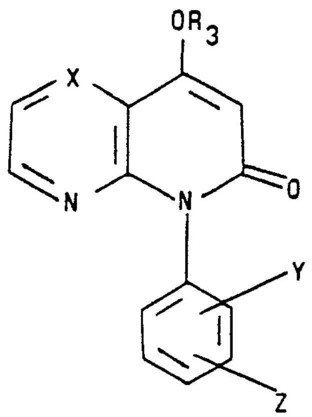

(IV)

dans laquelle $R_3$ est un groupe alcényle avec 3 à 6 atomes de carbone ayant une double liaison en position 2, 3 et X, Y et Z ont les significations définies ci-dessus, et en ce que un composé obtenu conformément au processus A ou B ci-dessus, dand laquel $R_2$ représente l'hydrogène, si on le désire, est soumis à l'introduction d'un groupe $R_2$ différent de l'hydrogène.

2. Procédé selon la revendication 1B, caractérisé en ce que le réarrangement de Claisen est effectué en présence d'un anhydride d'acide de mainère à obtenir un composé de la formule I dans lequel $R_1$ est un groupe alcényle avec 3 à 6 atomes de carbone ayant une double liaison en position 2,3 et $R_2$ est un groupe acyle.

3. Procédé selon la revendication 1A, caractérisé en ce qu'on prépare le sel de sodium, potassium, calcium, éthanolamine, N-méthylglucamine, diéthanolamine, éthylènediamine, tris-hydroxyéthyl-méthylamine ou lysine de 3-(n-butyl)-4-hydroxy-1-phényl-1,8-naphtyridine-2(1*H*)-one.

4. Procédé selon la revendiction 1, caractérisé en ce qu'on prépare un composé de la formule I dans laquelle Y signifie hydrogène, méthylthio ou méthoxy; Z signifie hydrogène ou méthyle; $R_1$ signifie n-$C_4H_9$, $-(CH_2)_3CH_2OCOC_2H_5$, $-CH_2CH_2OH$, $-(CH_2)_2CH_2OH$ ou $-CH_2-CH{=}CH_2$ et $R_2$ est un cation de sodium ou, à condition que $R_1$ ne soit pas n-$C_4H_9$, aussi de l'hydrogène, $-COCH_3$ ou $-COC_2H_5$.

5. Procédé selon la revendication 4, caractérisé en ce qu'on prépare 4-acétoxy-1-phényl-3-(2-propényl)-1,8-naphtyridine-2(1*H*)-one.

6. Procédé selon la revendiction 4, caractérisé en ce qu'on prépare 4-hydroxy-1-(3-méthoxyphényl)-3-(2-propényl)-1,8-naphtyridine-2(1*H*)-one ou le sel de sodium de celui-ci.

7. Procédé de préparation de compositions pharmaceutiques, caractérisé en ce qu'un composé tel que défini dans l'une quelconque des revendications 1 à 6 est mis en une forme convenant pour son administration en tant que produit pharmaceutique.